# EUROPÄISCHE PATENTSCHRIFT

(11) **EP 1 465 641 B1**
(45) Veröffentlichungstag und Bekanntmachung des Hinweises auf die Patenterteilung: **12.09.2007**
(21) Anmeldenummer: 02788259.6
(22) Anmeldetag: 12.12.2002
(51) Int. Cl.: A61K 33/00, A61P 3/10

(54) **ARZNEIMITTEL ENTHALTEND DEUTERIUM ZUR BEHANDLUNG DER ZUCKERKRANKHEIT**
PHARMACEUTICAL PRODUCTS COMPRISING DEUTERIUM FOR THE TREATMENT OF DIABETES
MEDICAMENTS CONTENANT DU DEUTERIUM POUR TRAITER LE DIABETE

(30) Priorität: 12.12.2001 HU 0105316
(43) Veröffentlichungstag der Anmeldung: 13.10.2004
(73) Patentinhaber: HYD Kutato.Fejleszto Kft, 1215 Budapest (HU)
(72) Erfinder: SOMLYAI, Gábor, H-1215 Budapest (HU)
(74) Vertreter: Palagyi, Tivadar
(86) Internationale Anmeldenummer: PCT/HU2002/000142
(87) Internationale Veröffentlichungsnummer: WO 2003/049748

(56) Entgegenhaltungen:
- WO-A-93/08794
- WO-A-95/18545
- WO-A-95/25526
- SOMLYAI G ET AL: "The biological effects of deuterium-depleted water, a possible new tool in cancer therapy" ZEITSCHRIFT FUR ONKOLOGIE 1998 GERMANY, Bd. 30, Nr. 4, 1998, Seiten 91-94, XP009006973 ISSN: 1432-2919 in der Anmeldung erwähnt

## Beschreibung

Die Erfindung betrifft Arzneimittel zur Behandlung der Zuckerkrankheit (Diabetes mellitus).

Als Zuckerkrankheit wird diejenige Krankheitsgruppe bezeichnet, bei der die Blutzuckerkonzentration des Kranken über dem Normalwert (3,5-6,6 mMol/l) liegt (Hyperglycaemia). Die zu der Krankheitsgruppe gehörenden Kranken haben trotz der unterschiedlichen Pathogenese eines gemeinsam: sie sind nicht in der Lage, die für ihren Stoffwechsel erforderliche Menge an Insulin zu produzieren.

Unter diagnostischem Aspekt können bei der Zuckerkrankheit drei Untergruppen unterschieden werden:
1. Die insulindependente Zuckerkrankheit (Typ 1) kommt im allgemeinen bei Kindern und jungen Erwachsenen vor, kann aber in jedem Lebensalter auftreten. Bei den zu dieser Gruppe gehörenden Kranken ist der endogene Insulinspiegel völlig verschwunden, daher ist es zur Verringerung des Blutzuckerspiegels, zum Überleben überhaupt zwingend, Insulin zu verabreichen.
2. Die nicht insulindependente Zuckerkrankheit (Typ 2) kommt im allgemeinen ab dem dreißigsten Jahr vor. Die Kranken sind im allgemeinen zu dick und insulin-resistent, was bedeutet, daß sie Insulin produzieren, aber bei ihnen die normale Menge Insulin eine subnormale Antwort auslöst, wodurch der Blutzuckerspiegel ansteigt. Zum Überleben ist exogenes Insulin nicht erforderlich.
3. Sonstige Formen der Zuckerkrankheit, die auf andere grundlegende Ursachen, zum Beispiel die Krankheit der Bauchspeicheldrüse, zurückzuführen sind.

Es ist bekannt, daß die Anzahl der Zuckerkranken 2-12 % der Einwohnerschaft eines Landes ausmachen kann. Wenn man die geographische Verteilung der Vorkommenshäufigkeit dieser Krankheit berücksichtigt, kann ein häufiges Vorkommen zum Teil mit zu starkem Essen, mit Übergewicht in Zusammenhang gebracht werden. Die die Krankheit auslösenden Gründe sind größtenteils bekannt, die Behandlung der Krankeit kann in gewissem Sinne als gelöst betrachtet werden, obwohl die fallweise sich über Jahrzehnte erstreckende Behandlung schwere Nebenwirkungen hat. Die sog. "akuten" Komplikationen (Hypoglycaemia, hyperosmotisches Koma, Milchsäureacidose) können zu jedem beliebigen Zeitpunkt manifest werden, und im Laufe der Jahre können Spätkomplikationen eintreten (Makroangiopathie, die sich in einem Anstieg der Häufigkeit atherosklerotischer Komplikationen zeigt; Mikroangiopathie, eine spezielle Schädigung der Kapillargefäße oder zum Beispiel eine gesteigerte Neigung zu Infektionen).

Obwohl die Krankheit behandelbar ist, zeigen die statistischen Daten, daß die Sterblichkeit unter den Zuckerkranken 2-3mal so hoch ist, Blindheit 10mal so oft auftritt, Absterben und Amputation von Gliedmaßen 20mal so häufig vorkommt, Krankenhausaufenthalt mehr als doppelt so oft erforderlich ist als in der Durchschnittsbevölkerung.

Ziel der Behandlung der Zuckerkrankheit ist es, die unmittelbaren Folgen des Insulinmangels abzuwehren und die Komplikationen der langfristig bestehenden Krankheit zu vermindern.

Die Behandlung der Zuckerkrankheit kann durch die gemeinsame Anwendung von drei unterschiedlichen Variablen optimiert werden: 1. spezielle Diät, 2. sorgfältige Planung körperlicher Arbeit, 3. Behandlung nit Arzneimitteln. Im Idealfall stehen die sorgfältig geplante Diät, der im Zuge körperlicher Arbeit auftretende Zuckerverbrauch und die sorgfältig dosierte Arzneimittelmenge miteinander in Einklang und gewährleisten den entsprechenden Blutzuckerspiegel mit den geringsten Schwankungen. Dies über längere Zeit hin zu erreichen gelingt leider nur in den seltensten Fällen, infolgedessen treten die bereits erwähnten Komplikationen auf.

In den vergangenen Jahren erschienen Publikationen [FEBS Lett. 317, 1-4 (1993); Természetgyógyászat 10, 29-32 (1996); Kisállatorvoslás 3, 114-5, (1996); Erfahrungsheilkunde 7, 381-88 (1997); J. R. Heys und D.G. Melillo (eds.), Synthesis and Applications of Isotopically Labelled Compounds, John Wiley and Sons Ltd., S. 137-141 (1997); Z. Onkol/J. of Oncol. 30: 91-94 (1998)] beziehungsweise ungarische Patentschriften (HU-208 084, HU-209 787) betreffend die Erkenntnis, daß das in der Natur vorkommende Deuterium in der Regulierung der Zellteilung eine wichtige Rolle spielt. Neben den in den Patentschriften beschriebenen Tierversuchen wurde die Antitumorwirkung des Wassers mit verringertem Deuteriumgehalt (Dd-Wasser) inzwischen auch durch Untersuchungen am Menschen nachgewiesen. In den letzten vier Jahren haben mehr als 800 Tumorkranke 100 Tonnen Dd-Wasser verbraucht. Die Fälle bestätigten, daß die Tumorzellen auf den Entzug des Deuteriums empfindlich reagieren und in den meisten Fällen (70-80 %) nicht fähig sind, sich den Änderungen anzupassen, was zur Folge hat, daß das Volumen des Tumors abnimmt bzw. der Tumor vollständig verschwindet.

Unter den Tumorkranken waren in den letzten Jahren auch viele Zuckerkranke. Die Kranken begannen wegen ihrer Tumorkrankheit mit der Einnahme des Dd-Wassers, jedoch erwies sich die Therapie überraschenderweise auch gegen die Zuckerkrankheit wirksam. Eigene Beobachtungen führten zu der unerwarteten Folgerung, daß sich die Verminderung der D-Konzentration günstig auf die Blutzuckerkonzentration im Organismus des Zuckerkranken auswirkt.

Aufgabe der Erfindung war daher die Entwicklung von Präparaten verringerten Deuteriumgehaltes, welche die Behandlung und Heilung der Zuckerkrankheit ermöglichen.

Grundlage der Erfindung ist die Erkenntnis, daß eine Verringerung der normalen Deuteriumkonzentration des Organismus (150 ppm, d. h. 16,8 mMol/l HDO-Konzentration) den Zuckerhaushalt des Organismus vorteilhaft beeinflußt, d. h. den schwankenden Blutzuckerspiegel im normalen Bereich oder in der Nähe des normalen Bereiches stabilisiert und den stabil hohen Blutzuckerspiegel senkt.

Eine weitere Grundlage der Erfindung ist die Erkenntnis, daß der Verzehr von Wasser, wäßrigen Lösungen mit einem D-Gehalt, der geringer ist als der natürliche, bzw. von in der Nahrung befindlichen Kohlehydraten, Aminosäuren und Lipiden, deren D-Gehalt geringer ist als der natürliche, den D-Gehalt des kranken Organismus verringert und dadurch eine Stabilisierung der schwankenden Blutzuckerkonzentration und eine Senkung der hohen Blutzuckerwerte ermöglicht.

Daher betrifft die Erfindung zur Behandlung und Heilung von Zuckerkranken geeignete, Wasser mit einem Deuteriumgehalt von 0,01-135 ppm, d.h. einem HDO-Gehalt von 0,021-287 mg/l, enthaltende Arzneimittelpräparate, ferner zur Behandlung und Heilung von Zuckerkranken geeignete, Wasser mit einem Deuteriumgehalt von 0,01-135 ppm, d.h. einem HDO-Gehalt von 0,021-287 mg/l, enthaltende Lebensmittelprodukte.

Gegenstand der Erfindung ist die Verwendung von 0,01-135 ppm Deuterium, d. h. 0,021-287 mg/l HDO, enthaltendem Wasser zur Herstellung von Arzneimittelpräparaten, die zur Behandlung und Heilung der Zuckerkrankheit geeignet sind.

Von den Methoden zur Herstellung von Wasser verringerten Deuteriumgehaltes sollen hier die Elektrolyse und die Destillation erwähnt werden. Mit diesen Verfahren kann Dd-Wasser in großen Mengen mit relativ geringen spezifischen Kosten hergestellt werden.
a) Eine 15-20 %ige wäßrige Kalilauge wird mit einer Gleichspannung von 2-5 V elektrolysiert, wobei die Kathode von der Anode getrennt ist. Der sich an der Kathode entwickelnde, an D verarmte Wasserstoff wird verbrannt, der entstehende Wasserdampf wird kondensiert und getrennt gesammelt. Bei diesem Verfahren entsteht Wasser mit einer D-Konzentration von 30-40 ppm (Separation of Hydrogen Isotopes, Eds.: Howard K. Rae, American Chemical Society Symposium Series 68, Washington D. C. 1978; Isotope Separations, Eds.: Stelio Villani, American Nuclear Society, 1983). Der D-Gehalt des auf diese Weise erhaltenen Wassers kann durch wiederholte Elektrolyse weiter gesenkt werden.
b) Destilliertes Wasser wird in einer Fraktionskolonne mit 30-50 Böden unter einem Druck von 5-6 kPa bei 45-50 °C gekocht. Während der Destillation beträgt der Refluxwert 12-13, der Sumpfrücklauf ist zehnfach. Bei Anwendung dieser Parameter kann als Kopfprodukt Wasser mit einer D-Konzentration von 0,1-30 ppm erhalten werden (Separation of Hydrogen Isotopes, Eds.: Howard K. Rae, American Chemical Society Symposium Series 68, Washington D. C., 1978; Isotope Separations, Eds.: Stelio Villani, American Nuclear Society, 1983). Werden während des Betriebes der Kolonne die Parameter geändert, wird zum Beispiel die Belastung der Kolonne erheblich vergrößert, so besteht die Möglichkeit, in großen Mengen auch Wasser mit einem D-Gehalt von mehr als 30 ppm herzustellen. Eine weitere Möglichkeit besteht darin, das von der Kolonne gewonnene Wasser verringerten D-Gehaltes in einer nachgeschalteten, ähnlichen Kolonne einer fraktionierten Destillation zu unterziehen und auf diese Weise seinen D-Gehalt weiter zu verringern.

Kohlehydrate, Aminosäuren und Lipide verringerten D-Gehaltes können zweckmäßig hergestellt werden, indem man 0,01-135 ppm Deuterium, d. h. 0,021-287 mg/l HDO, enthaltendes Wasser in an sich bekannter Weise im Pflanzenanbau und in der Tierzucht verwendet.

Das Dd-Wasser dient als Ausgangsstoff zur Herstellung von zur Heilung der Zuckerkrankheit geeigneten Präparaten.

Die mit dem erfindungsgemäßen Verfahren hergestellten Präparate sind zur Heilung von Zuckerkranken geeignet. Grundlage dafür ist, daß durch die Aufnahme von mit Dd-Wasser bereiteten Lösungen und/oder Lebensmitteln, Kohlehydraten, Aminosäuren und Lipiden verminderten D-Gehaltes die D-Konzentration im Organismus abnimmt, wodurch die Möglichkeit entsteht, den schwankenden Zuckerspiegel zu stabilisieren und die hohen Blutzuckerwerte zu senken.

Die Eignung der Erfindung zur Heilung von Zuckerkranken wurde durch die Ergebnisse von Versuchen bestätigt, in denen Menschen mit Dd-Wasser behandelt wurden.

V. K.: 1993 schwankte der Blutzuckerspiegel des 67jährigen männlichen Patienten zwischen 6,9 und 12 mMol/l. Durch Verabreichung von täglich 0,5 Liter 90 ppm Deuterium enthaltendem Dd-Wasser stabilisierte sich die Blutzuckerkonzentration innerhalb von zwei Monaten bei 8-9 mMol/l.

Z. P.: 1994 wurde bei der 52jährigen Patientin bei Aufstellung der Diagnose ein Blutzuckerwert von 14 mMol/l gefunden. Die Patientin erhielt keine der herkömmlich bei Zuckerkranken angewendeten Behandlungen. Durch die Einnahme von Dd-Wasser (täglich 0,5-0,7 Liter 90 ppm Deuterium enthaltendes Dd-Wasser) sank ihr Blutzuckerspiegel innerhalb eines Monats auf 8 mMol/1.

K. Cs.: Die 1936 geborene Patientin wurde 1994 zuckerkrank infolge der Hormonpräparate, die sie wegen eines 7 Jahre vorher diagnostizierten Brusttumors nahm. Ihre Blutzuckerwerte schwankten, der maximale Wert betrug 9,7 mMol/l, der Durchschnittswert 7,9 mMol/l. Durch die Verabreichung von täglich 1,4 Liter Dd-Wasser mit einem D-Gehalt von 100 ppm stabilisierte sich der Blutzuckerwert bei 4,5 mMol/l, die extrem hohen Werte verschwanden.

O. F.: Die Blutzuckerkonzentration des 1994 76jährigen Mannes betrug stabil 12-14 mMol/l. Der Patient erhielt zuerst täglich 0,8 Liter Dd-Wasser mit 100 ppm, dann täglich 1 Liter mit 90 ppm. Der Blutzuckerspiegel sank nach einem Monat der Kur zuerst auf 10,2 mMol/l und nach zwei weiteren Wochen auf 7,4 mMol/l.

H. A.: Die Zuckerkrankheit des 1994 62jährigen wurde 1990 diagnostiziert. Durch die Behandlung mit Arzneimitteln bewegte sich der Blutzuckerspiegel stabil zwischen 16 und 17 mMol/l. Im Januar 1995, nach einer dreimonatigen Kur mit Dd-Wasser (1,2 Liter, D-Gehalt 90 ppm) sank der Blutzuckerspiegel auf 10,2 mMol/l. Ein Jahr später, im Januar 1996, wurden 7,6-7,9 mMol/l Blutzucker gemessen. Gegen Ende des Jahres sank der Blutzuckerwert weiter (7,3 mMol/l), obwohl die Arzneimitteldosis des Patienten auf die Hälfte des ursprünglichen Wertes gesenkt wurde.

N. S.: An dem 1987 geborenen Knaben wurde 1992 die Zuckerkrankheit diagnostiziert. Vor der Dd-Wasserkur erhielt das Kind (27 kg Körpergewicht) morgens 2+3 Einheiten und abends 2+2 Einheiten Insulin. Der Blutzucker schwankte zwischen den Extremwerten 17,5 und 1,9 mMol/l. Nach zwei Monaten, in denen täglich 0,4 Liter Dd-Wasser (90 ppm) verabreicht wurde, verschwanden die unteren Extremwerte, der Blutzuckerspiegel sank nicht unter 5 mMol/l, die oberen Werte waren nicht höher als 13,5 mMol/l.

Z. G.: An dem 1979 geborenen Knaben wurde im September 1996 ein Blutzuckerspiegel von 9,8-15,5 mMol/l gemessen und anschließend die Zuckerkrankheit diagnostiziert. Der Patient erhielt vom Oktober 1996 bis zum Mai 1998 täglich 0,5 Liter Dd-Wasser (90 ppm). In diesem Zeitabschnitt bewegten sich die Blutzuckerwerte des Patienten zwischen 5 und 7 mMol/l, eine Insulingabe war nicht erforderlich. Einige Wochen nach Absetzen der Dd-Wasserbehandlung wurden bei der Kontrolluntersuchung 14-20 mMol/l Blutzuckerspiegel gemessen. Im Oktober 1998 begann der Patient erneut, Dd-Wasser zu trinken, danach stellte sich zum Januar 1999 der frühere Blutzuckerwert wieder ein.

Die Ergebnisse der bisher an Menschen vorgenommenen Versuche zusammenfassend kann gesagt werden, daß der Verbrauch von Wasser verringerten Deuteriumgehaltes im Fall von Zuckerkranken den Blutzuckerspiegel des Kranken günstig beeinflußt. Dadurch konnte fallweise erzielt werden, daß die Dosis des herkömmlichen Arzneimittels verringert werden konnte.

Die erfindungsgemäßen Präparate können den Wirkstoff und inerte, nichttoxische Trägerstoffe enthalten. Der Wirkstoff kann zu oral verabreichbaren Präparaten (zum Beispiel Lösung, Emulsion, Suspension usw.) oder zu parenteral applizierbaren Präparaten (zum Beispiel Infusionslösung) formuliert werden.

Die Herstellung der Arzneimittelpräparate erfolgt auf die in der Arzneimittelindustrie üblichen Weise, indem der Wirkstoff und der inerte anorganische oder organische Trägerstoff miteinander vermischt werden und das Gemisch in eine galenische Form gebracht wird.

Als Trägerstoff wird bevorzugt Wasser verwendet.

Die Arzneimittelpräparate können ferner weitere, in der Arzneimittelindustrie übliche Hilfsstoffe (zum Beispiel Netzmittel, Süßstoffe, Aromastoffe, Pufferlösungen usw.) enthalten.

Die tägliche Dosis der erfindungsgemäßen Arzneimittelpräparate kann innerhalb eines weiten Bereiches variieren und hängt von verschiedenen Faktoren ab, zum Beispiel von der D-Konzentration des Wassers, von Gewicht und Alter des Patienten, dem Typ und der Schwere der Zuckerkrankheit usw. Für einen Kranken von 70 kg Körpergewicht kann die tägliche Dosis 0,01-2 Liter D-Wasser betragen, dessen D-Konzentration zwischen 0,01 und 135 ppm liegt. Zum Teil, um den Genußwert, zum Teil, um die Wirkung zu erhöhen, kann das Wasser zum Beispiel 20-30 g Kohlehydrat verminderten D-Gehaltes oder gewisse Aminosäuren verminderten D-Gehaltes beziehungsweise sonstige Geschmacks- und Aromastoffe enthalten.

Die erfindungsgemäßen Präparate und das erfindungsgemäße Verfahren haben die folgenden Hauptvorteile:
a) Durch ihre Anwendung eröffnet sich die Möglichkeit, in ein bisher unbekanntes Regulationssystem der Zelle einzugreifen.
b) Die Regulierung des Blutzuckerspiegels der Kranken wird ermöglicht.
c) Durch Anwendung der erfindungsgemäßen Präparate kann die Dosis herkömmlicher Arzneimittel gesenkt bzw. können diese ganz weggelassen werden.
d) Es ist gewährleistet, daß die Blutzuckerkonzentration innerhalb des physiologischen Bereiches bleibt, dadurch wird die Wahrscheinlichkeit der Herausbildung früher beziehungsweise später Komplikationen verringert.
e) Die in dem Verfahren verwendeten Verbindungen haben keine toxischen Nebenwirkungen.
f) Die Herstellung der Präparate ist einfach, es entstehen keine schädlichen Abfälle.

Das Verfahren wird an Hand der folgenden Ausführungsbeispiele erläutert.

### Beispiel 1 (nicht im Rahmen der Erfindung)

### Herstellung von Trinkwasser mit einer günstigen Salzzusammensetzung

Das Wasser mit verringertem D-Gehalt wird mit einem Mineralwasser bekannter Salzzusammensetzung (Csillaghegyi, Balfi) in folgenden Verhältnissen gemischt:
a) 0,25 Volumenteile 90 ppm D enthaltendes Wasser + 0,75 Volumenteile Mineralwasser (Endkonzentration an D 135 ppm)
b) 0,5 Volumenteile 90 ppm D enthaltendes Wasser + 0,5 Volumenteile Mineralwasser (Endkonzentration an D 120 ppm)
c) 0,75 Volumenteile 90 ppm D enthaltendes Wasser + 0,25 Volumenteile Mineralwasser (Endkonzentration an D 105 ppm)
d) 0,25 Volumenteile 60 ppm D enthaltendes Wasser + 0,75 Volumenteile Mineralwasser (Endkonzentration an D 127,5 ppm)
e) 0,5 Volumenteile 60 ppm D enthaltendes Wasser + 0,5 Volumenteile Mineralwasser (Endkonzentration an D 105 ppm)

### Beispiel 2 (nicht im Rahmen der Erfindung)

Die Kationen- und Anionenkonzentration des Wassers mit verringertem D-Gehalt wird mit einem künstlich hergestellten Konzentrat günstiger Salzzusammensetzung eingestellt. Eine mögliche Zusammensetzung der Stammlösung ist folgende:

| | |
|---|---|
| KCl | 5,7 g |
| MgCl₂ x 6 H₂O | 199,65 g |
| CaCl₂ x 6 H₂O | 236,25 g. |

Wird die auf diese Weise hergestellte Stammlösung zu 1000 Liter Dd-Wasser gegeben, so stellen sich die folgenden Endkonzentrationen ein:
23,8 mg/l Mg²⁺, 64,1 mg/l Ca²⁺, 3 mg/l K⁺, 192 mg/l Cl⁻.

### Beispiel 3 (nicht im Rahmen der Erfindung)

### Herstellung von Lebensmitteln mit verringertem D-Gehalt

Unter Verwendung des 0,01-135 ppm D, d. h. 0,021-287 mg/l HDO, enthaltenden Wassers werden in bekannter Weise Paprika, Tomaten, Schoten und grüne Bohnen angebaut. Die Pflanzen werden auf die in der Lebensmittelindustrie übliche Weise zu Lebensmitteln aufbereitet.

### Beispiel 4 (nicht im Rahmen der Erfindung)

### Herstellung von Kohlehydraten (Zuckern) verringerten Deuteriumgehaltes

Das 0,01-135 ppm D, d. h. 0,021-287 mg/l HDO, enthaltende Wasser wurde unter Gewächshausverhältnissen zum Gießen von zuckerreichen Zuckerrüben verwendet. Aus den auf diese Weise herangezogenen Rüben wird der Zucker verminderten D-Gehaltes mit dem bei der Aufarbeitung von Zuckerrüben üblichen Verfahren gewonnen.

### Beispiel 5 (nicht im Rahmen der Erfindung)

### Herstellung von Eiweiß verringerten D-Gehaltes

Das 0,01-135 ppm D, d. h. 0,021-287 mg/l HDO, enthaltende Wasser wurde unter Gewächshausverhältnissen zum Gießen von eiweißreicher Soja verwendet. Die Aufarbeitung der Pflanzen erfolgt mit den in der Lebens- und Futtermittelindustrie üblichen Verfahren.

### Beispiel 6 (nicht im Rahmen der Erfindung)

### Herstellung von Lipiden, Ölen verringerten Deuteriumgehaltes

Das 0,01-135 ppm D, d. h. 0,021-287 mg/l HDO, enthaltende Wasser wurde unter Gewächshausverhältnissen zum Gießen von ölreichen Sonnenblumen verwendet. Die Aufarbeitung der Pflanzen erfolgt mit den in der Lebens- und Futtermittelindustrie üblichen Verfahren.

### Beispiel 7 (nicht im Rahmen der Erfindung)

### Herstellung von eiweiß- und lipidreichen Lebensmitteln verringerten Deuteriumgehaltes

Die unter Verwendung des 0,01-135 ppm D, d. h. 0,021-287 mg/l HDO, enthaltende Wassers zum Gießen angebauten Pflanzen werden auf die in der Futtermittelindustrie übliche Weise aufgearbeitet. Das auf diese Weise hergestellte Futter verringerten Deuteriumgehaltes wird zum Füttern der in der Landwirtschaft gehaltenen Tiere verwendet, gleichzeitig wird das Trinkwasser der Tiere durch 0,01-135 ppm D, d. h. 0,021-287 mg/l HDO, enthaltendes Wasser ersetzt. Die geschlachteten Tiere, die Eiweiße verringerten D-Gehaltes enthalten, werden auf die in der Lebensmittelindustrie übliche Weise verarbeitet.

## Patentansprüche

1. Verwendung von 0,01-135 ppm Deuterium, d. h. 0,021-287 mg/l HDO, enthaltendem Wasser zur Herstellung von zur Behandlung und Heilung der Zuckerkrankheit geeigneten Arzneimittelpräparaten.

## Claims

1. Use of water containing 0.01-135 ppm of deuterium, that is 0.021-287 mg/l of HDO, for preparing pharmaceutical preparations useable for treating and curing diabetes.

## Revendications

1. Emploi de l'eau contenant 0,01-135 ppm de deutérium, c'est-à-dire 0,021-287 mg/l de HDO, pour manufacturer de préparations pharmaceutiques propre à traiter et guérier le diabète.
